# EUROPEAN PATENT SPECIFICATION

(11) **EP 1 849 466 B1**
(45) Date of publication and mention of the grant of the patent: **18.01.2012**
(21) Application number: 07251707.1
(22) Date of filing: 24.04.2007
(51) Int. Cl.: A61K 31/195, A61K 31/198, A61K 31/401, A61K 31/405, A61K 9/08

(54) **Infusion fluid for treating anemia in dialysis patients**
Infusionsflüssigkeit zur Behandlung der Anämie bei Dialysepatienten
Liquide d'infusion pour le traitement de l'anémie chez les patients sous dialyse

(30) Priority: 24.04.2006 JP 2006119694; 27.04.2006 JP 2006124235; 21.06.2006 JP 2006171886
(43) Date of publication of application: 31.10.2007
(73) Proprietor: Ajinomoto Co., Ltd., Tokyo 104-8315 (JP)
(72) Inventor: Sugiyama, Takayuki, Tokyo 104-8315 (JP)
(74) Representative: Nicholls, Kathryn Margaret

(56) References cited:
- US-A1- 2002 144 946
- US-A1- 2005 148 647
- US-B1- 6 830 692
- NAVARRO JUAN F ET AL: "Amino acid losses during hemodialysis with polyacrylonitrile membranes: Effect of intradialytic amino acid supplementation on plasma amino acid concentrations and nutritional variables in nondiabetic patients" AMERICAN JOURNAL OF CLINICAL NUTRITION, vol. 71, no. 3, March 2000 (2000-03), pages 765-773, XP002445740 ISSN: 0002-9165

## Description

The present invention relates to an infusion fluid for dialysis patients and in particular, to a treatment for anemia in dialysis patients receiving hemodialysis, hemodiafiltration or other hemodialytic treatments. The present invention may also be used in a method for ameliorating anemia in patients with chronic renal failure. It may reduce the amount of erythropoietin (EPO), a hematopoietic hormone administered to chronic renal failure patients to treat anemia, which is required.

Hemodialysis (HD) is one of the blood purification techniques used to treat patients with chronic renal failure. One problem with hemodialysis is that it removes not only waste products, but also useful substances such as amino acids, from the body of a patient. Recent dialysis techniques adapted to long-term treatment are often associated with a condition known as dialysis-related amyloidosis, which develops as a result of the accumulation of a relatively large protein, beta-2 microglobulin (MW = 11800). To prevent amyloidosis, many of these techniques involve the use of high-performance membranes for removing beta-2 microglobulin. However, high-performance membranes can remove serum albumin (MW = several tens of thousands), along with the microglobulin. As a result, the patients may suffer from iatrogenically caused malnutrition.

As albumin and amino acids are continually lost during dialysis, myolysis, or protein catabolism, occurs. The protein catabolism is more significant in hemodialysis (HD) than in hemodiafiltration (HFD which is performed to positively remove dissolved substances).

Malnutrition adversely affects the prognosis of patients, and improving nutritional status in dialysis patients is a highly important issue.

With rare exceptions, chronic renal failure is associated with anemia (specifically called "renal anemia"). Patients with severe anemia can easily become short of breath while walking a short distance. Severe renal anemia can also lead to decreased activities of daily living (ADL) and cause heart failure and other secondary complications. Therefore, renal anemia is a serious problem for dialysis patients.

The most important cause of anemia in dialysis patients is the defective production of a hematopoietic hormone erythropoietin (EPO). Most of the patients currently receiving dialysis also receive EPO to treat their anemia. Dialysis patients with malnutrition generally show a poor response to EPO. In other words, normal doses of EPO do not have sufficient effect on anemia in these patients. Thus, dialysis patients with malnutrition often require EPO administered in large doses.

Recent studies report that EPO treatment of anemia in dialysis patients ameliorates not only anemia itself, but also nutritional status of the patients (See, for example, Non-Patent Documents 1 through 6). This observation suggests that the EPO treatment for anemia might be responsible for the improvement in nutritional status.

As described above, serum albumin and amino acids are rapidly removed from the blood of patients during dialysis. Such drastic changes, which are not normally experienced by healthy people, cause severe malnutrition in dialysis patients during dialysis. Metabolic acidosis, renal anemia, inflammation and other symptoms of patients with renal failure can also cause catabolism of amino acids and proteins, leading to malnutrition.

One effective approach to avoid such rapid changes in the blood is the continuous infusion of amino acids, which quickly replaces amino acids lost during dialysis. In dialysis patients who suffer from amino acid depletion of the sort that is never experienced by healthy people, it is extremely important to replace the amino acids lost during dialysis. The replacement of the lost amino acids can prevent or alleviate malnutrition or protein catabolism during dialysis.

From that point of view, improving the nutritional status in dialysis patients by replacing the amino acids, especially essential amino acids, is considered the most effective way to ameliorate anemia in such patients. Such an approach to ameliorate anemia can decrease the dose of EPO administered to dialysis patients.

While various amino acid preparations have been developed for use in patients with renal failure, no literatures or reports exist that describe the use of these drugs to ameliorate malnutrition-associated anemia in dialysis patients or the advantage of using these drugs to reduce the dose of EPO.

Hyperphosphatemia is generally defined as a condition in which the serum phosphate level is elevated to 5.0 mg/dL or higher. Hyperphosphatemia is caused by (i) increased phosphate released from the cells, (ii) increased phosphate intake or (iii) decreased renal phosphate excretion. Serum phosphate levels also increase when the glomerular filtration rate (GFR) is decreased to 30% or less of the normal rate due to defective renal functions such as renal failure.

Thus, patients with renal failure tend to have high serum phosphate levels and are recommended to avoid phosphorus-rich food products such as meat and milk. This also facilitates the reduction of albumin and amino acids, thus resulting in significant protein catabolism.

An increase in the phosphate levels causes binding of phosphate to calcium in blood, which can lead to ectopic calcification, a major cause of arteriosclerosis.

In addition to diet, phosphate binders (such as sevelamer hydrochloride and precipitated calcium carbonate (Non-Patent Document 7) are currently used to prevent elevation of serum phosphate levels. However, decreasing the blood phosphate levels is considered merely a symptomatic treatment and regarded as a last resort since phosphate is not adequately utilized in myolysis and dysostosis. Therefore, a desirable treatment is to make phosphate available by constantly controlling the serum phosphate levels within an optimum range, rather than to simply decrease the phosphate levels.

Preferably, a phosphate binder for treating hyperphosphatemia in renal failure patients receiving dialysis is administered at a particular dose so that the serum phosphate level is kept below 6.0 mg/L. The dose is adjusted depending on the serum phosphate level. However, as described above, the adjustment of serum phosphate levels by phosphate binders is only a symptomatic treatment and regarded as a last resort.

As described above, serum albumin and amino acids are rapidly removed from the blood of patients during dialysis. As a result, dialysis patients will experience severe protein catabolism. To prevent this, the loss of amino acids is compensated for by continuous infusion of amino acids, which quickly replaces the amino acids lost during dialysis. Replenishing amino acids prevents or alleviates malnutrition or protein catabolism during dialysis, thereby suppressing the elevation of serum phosphate levels due to myolysis. In this manner, the serum phosphate levels can be controlled.

While various amino acid preparations have become available for use in patients with renal failure, no literatures or reports exist that describe the use of these drugs to control the serum phosphate levels in dialysis patients within an optimum range.
Non-Patent Document 1: Gunnell J., et al.: Am. J. Kid. Dis., 33, 63-71 (1999)
Non-Patent Document 2: Balaskaa E. V., et al.: Miner Electrolyte Metab., 25, 324-332 (1999)
Non-Patent Document 3: Navarro J. F., et al.: Am. J. Clin. Nutr., 71, 765-773 (2000)
Non-Patent Document 4: Serna-Thome M. G., et al.: Curr. Opinion Clin. Nutr. Metab. Care 5, 293-296 (2002)
Non-Patent Document 5: Pupim L. B., et al.: J. Clin. Invest., 110, 483-492 (2002)
Non-Patent Document 6: Pupim L. B., et al.: J. Am. Soc. Nephrol., 15, 1920-1926 (2004)
Non-Patent Document 7: Japanese Pharmacopoeia 14th ed., Paragraphs regarding precipitated calcium carbonate

Embodiments of the invention are described below, by way of example only, and with reference to the accompanying drawings, of which:
Fig. 1 is a graph showing changes in the dose of EPO according to Example 2.
Fig. 2 is a graph showing changes in the hemoglobin (Hb) concentration according to Example 2.
Fig. 3 is a graph showing changes in the hematocrit (Ht) according to Example 2.
Fig. 4 is a graph showing changes in the serum phosphate level (for individual patients) according to Example 3.
Fig. 5 is a graph showing changes in the 3-m-His concentration in plasma according to Example 4.
Fig. 6 is a graph showing changes in the 3-m-His concentration in dialysate according to Example 4.
Fig. 7 is a graph showing changes in the taurine concentration in plasma according to Example 4.
Fig. 8 is a graph showing changes in the taurine concentration in dialysate according to Example 4.
Fig. 9 shows a comparison of the total amino acid concentrations in plasma according to Example 5.
Fig. 10 shows a comparison of the concentrations of essential amino acids in plasma according to Example 5.
Fig. 11 shows a comparison of the concentrations of non-essential amino acids in plasma according to Example 5.
Fig. 12 shows a comparison of the concentrations of total amino acids, essential amino acids and non-essential amino acids in dialysate according to Example 5.
Fig. 13 is an aminogram of essential amino acids prior to administration (0W) according to Example 5.
Fig. 14 is an aminogram of essential amino acids 37 weeks after administration (37W) according to Example 5.
Fig. 15 is an aminogram of non-essential amino acids prior to administration (0W) according to Example 5.
Fig. 16 is an aminogram of non-essential amino acids 37 weeks after administration (37W) according to Example 5.

In view of the above-described state of the art, it is an object of the present invention to provide an infusion fluid for use in treating anemia in dialysis patients. It may also serve for reducing the dose of erythropoietin administered to dialysis patients.

It may also serve for controlling serum phosphate levels, e.g. in patients with chronic renal failure receiving hemodialysis. Embodiments of the pharmaceutical preparation desirably control the serum phosphate levels within a specific range by ameliorating malnutrition in patients.

It may also serve for suppressing protein catabolism, e.g. in patients with chronic renal failure receiving hemodialysis.

In the course of studies to achieve the above-described objects of the invention, the present inventor has found that the continuous infusion of an amino acid solution formulated to replace amino acids lost during hemodialysis alleviates anemia in dialysis patients and, as a result, the dose of erythropoietin can be effectively reduced.

The present inventor has also found that the continuous infusion of such an amino acid solution ameliorates nutritional status of dialysis patients, so that the serum phosphate levels in the patients can be controlled within an optimum range. These findings have ultimately led to the present invention.

It is known that taurine is normally present at considerably high concentrations in muscles and at low concentrations in blood: The ratio of taurine concentration in muscles to that in plasma is normally 300 or higher. However, advanced myolysis or protein catabolism causes a release of taurine into blood, resulting in high plasma taurine levels. The present inventor has discovered that the plasma taurine levels can be used as an index of myolysis and protein catabolism and the continuous infusion of the above-described amino acid solution can be carried out based on this index in order to ameliorate protein catabolism in dialysis patients.

Accordingly, a first aspect of the present invention concerns an infusion fluid for use in treating anemia in patients receiving dialysis, wherein the infusion fluid contains at least one essential amino acid, and is administered by continuous infusion.

A more specific embodiment of the first aspect, the invention of claim 2 comprises the infusion fluid according to claim 1 which has an amino acid composition comprising at least L-isoleucine, L-leucine, L-lysine, L-methionine, L-phenylalanine, L-threonine, L-tryptophan, L-valine, L-alanine, L-arginine, L-aspartic acid, L-glutamic acid, L-histidine, L-proline, L-serine, L-tyrosine, glycine and L-cysteine with the ratio of essential amino acids to non-essential amino acids being 2.5 or higher.

In the invention of claim 3, a most specific embodiment of the first aspect, the amino acid infusion fluid has the following amino acid composition:

| | |
|---|---|
| L-isoleucine | 5.0 - 10.0 g/L |
| L-leucine | 8.0 - 12.0 g/L |
| L-lysine (as acetate) | 4.5 - 10.5 g/L |
| L-methionine | 3.5 - 6.5 g/L |
| L-phenylalanine | 3.5 - 6.5 g/L |
| L-threonine | 1.8 - 3.3 g/L |
| L-tryptophan | 1.8 - 3.3 g/L |
| L-valine | 5.0 - 10.0 g/L |
| L-alanine | 1.5 - 4.5 g/L |
| L-arginine | 1.5 - 4.5 g/L |
| L-aspartic acid | 0.2 - 0.4 g/L |
| L-glutamic acid | 0.2 - 0.4 g/L |
| L-histidine | 1.8 - 3.3 g/L |
| L-proline | 1.0 - 3.0 g/L |
| L-serine | 0.5 - 1.5 g/L |
| L-tyrosine | 0.3 - 0.7 g/L |
| glycine | 1.2 - 1.7 g/L |
| L-cysteine | 0.2 - 0.4 g/L. |

Preferably, the infusion fluid according to the first aspect of the present invention is continuously infused by means of a pump into the vein side of a dialysis circuit.

As set out in claim 5, the infusion fluid containing at least one essential amino acid may also serve for reducing the doseof EPO. The amino acid infusion fluid suitably has the amino acid composition according to claim 2, and preferably has the amino acid composition according to claim 3.

As set out in claim 6, the infusion fluid may also serve for controlling serum phosphate levels in patients receiving dialysis.

The amino acid infusion fluid of claim 6 preferably has the amino acid composition according to claim 2, and more preferably has the amino acid composition according to claim 3.

Serum phosphate levels in dialysis patients may thus be controlled within a range of 3.5 to 6.0 mg/dL.

The present invention may be used in a method for controlling serum phosphate blood levels in patients receiving dialysis, comprising administering an infusion fluid embodying the invention to patients with renal failure.

As set out in claim 7, the infusion fluid may also be used for suppressing protein catabolism in patients receiving dialysis least one essential amino acid.

Preferably the amino acid infusion fluid has the amino acid composition according to claim 2, and more preferably it has the amino acid composition according to claim 3.

The infusion fluid of the present invention can serve to replace or replenish amino acids lost during hemodialysis in patients with chronic renal failure and suppress protein degradation or protein catabolism in these patients, thereby maintaining and ameliorating the nutritional status of the patients. As a result, anemia in these patients can be alleviated.

It turned out that, in practice, the blood hemoglobin (Hb) level was maintained at 10 to 11 g/dL and the hematocrit (Ht) was maintained at about 30 to 33% by the use of the invention.

This observation, together with the fact that erythropoietin is generally administered to a patient with a blood Hb level of 10 g/dL or below or an Ht of less than 30%, suggests that the present invention is useful for effectively alleviating anemia in dialysis patients.

The amelioration of anemia leads to a reduction in the dose of erythropoietin administered during dialysis, which in turn decreases the medication cost that patients with chronic renal failure have to pay to receive hemodialysis. This is particularly advantageous in countries like Japan where the dialysis cost specified by the health insurance system includes medication cost because the patients can receive more sophisticated dialysis therapies under such insurance system.

The reduction in the dose of erythropoietin provides another benefit to patients because the frequency of side effects can be decreased, making dialysis a safer treatment.

Likewise, the infusion fluid serves to replace amino acids lost during hemodialysis in patients with chronic renal failure and suppress protein degradation or protein catabolism in these patients. This helps maintain and improve the nutritional status of the patients. As a result, the serum phosphate levels in these patients can be controlled within a predetermined range. Specifically, the serum phosphate levels in patients with chronic renal failure can be decreased to fall within a predetermined range if they are too high or maintained within that range if they are already in the range.

This significantly reduces the risk of complications in hemodialysis patients with chronic renal failure. As a result, the quality of life (QOL) of the patients can be improved, as can the prognosis of the patients.

It is known that each of the existing treatments for hyperphosphatemia has its own side effects. For example, precipitated calcium carbonate causes hypercalcemia, whereas sevelamer hydrochloride causes constipation, abdominal distension and other gastrointestinal symptoms and chloride acidosis. Due to these side effects, the traditional treatments for hyperphosphatemia cannot be used in doses high enough to lower the serum phosphate levels to the desired level. In particular, patients with significantly high serum phosphate levels require correspondingly high doses of the treatments, which result in an increased risk of side effects. Thus, these patients often receive insufficient doses of the treatments.

While some of the incidences of abnormally high serum phosphate levels are caused by excessive consumption of phosphate-rich food, such as meat, others are considered to be caused by protein catabolism, or dysostosis. The infusion fluid of the present invention for controlling serum phosphate levels can be used in conjunction with traditional treatments for hyperphosphatemia to compensate for their insufficient effects.

It is known that weakened muscles, decreased cardiovascular functions and decreased QOL will result when hemodialysis-associated protein catabolism is chronically repeated three times a week. The infusion fluid of the present invention can be used to suppress protein catabolism, thereby preventing or alleviating these symptoms in patients receiving long-term hemodialysis.

The amino acid infusion fluid, a pharmaceutical preparation provided in accordance with the present invention, desirably contains at least the following eight essential amino acids: L-isoleucine, L-leucine, L-lysine, L-methionine, L-phenylalanine, L-threonine, L-tryptophan and L-valine. When the infusion fluid is intended for use in patients with renal failure, it desirably further contains L-histidine, which is also essential to these patients. The infusion fluid may contain additional amino acids, including L-alanine, L-arginine, L-aspartic acid, L-glutamic acid, L-proline, L-serine, L-tyrosine, glycine and L-cysteine.

These amino acids are not necessarily used in the form of free amino acids: They may be used in the form of pharmaceutically or dietary acceptable salts. For example, they may be used in the form of pharmaceutically or dietary acceptable inorganic acid salts, organic acid salts, hydrolyzable esters or N-acyl derivatives. They may also be used in the form of peptides consisting of the same or different amino acids linked by peptide bonds.

The amino acid infusion fluid of the present invention is preferably formulated so that it is rich in essential amino acids. Specifically, the infusion fluid is formulated such that the ratio of essential amino acids to non-essential amino acids is 2.5 or higher, preferably 3.0 or higher, and most preferably 3.2 or higher.

The amino acid infusion fluid of the present invention desirably contains amino acids in amounts that can effectively replace amino acids and nutrients lost during hemodialysis. For example, 200 ml of the infusion fluid may contain 11 g to 13 g of amino acids in total with about 70% or more being essential amino acids. Preferably, the infusion fluid contains amino acids at a concentration of about 5.5 to about 6.5 w/v%.

The amino acid infusion fluid of the present invention may further contain electrolytes. Examples include potassium ions, calcium ions, magnesium ions, chloride ions, bicarbonate ions and gluconic acid ions. The infusion fluid may further contain lactic acid, citric acid and malic acid and may be made free of acetic acid ions to avoid acetate intolerance.

The amino acid infusion fluid of the present invention may be continuously infused through central vein lines by total parenteral nutrition technique. Preferably, the infusion fluid is continuously infused, along with a dialysate, over the course of dialysis.

Specifically, the infusion fluid of the present invention is continuously infused over the entire course of dialysis (typically about 4 hours) as opposed to traditional amino acid infusion fluids for renal failure, which are typically started 90 to 60 minutes before the end of dialysis and terminated at the end of dialysis. This is because albumin and amino acids start to be lost at the beginning of dialysis.

Preferably, the infusion fluid is continuously infused into the dialysis circuit that returns the dialyzed blood to the body by applying pressure with a pump. Specifically, the infusion fluid pressurized with a pump is continuously infused into the vein side of the dialysis circuit over the entire course of dialysis for at least a three-month period.

Most preferably, the amino acid infusion fluid of the present invention that serves as a pharmaceutical preparation for treating a variety of conditions in dialysis patients contains total nitrogen of 8.1 mg/mL and total free amino acids at a concentration of 5.90 w/v% with the ratio of essential amino acids to non-essential amino acids being 3.21.

Preferably, the amino acid infusion fluid suitable for use in the present invention has the following amino acid composition (in 200 mL):

| | |
|---|---|
| L-isoleucine | 1.5 g |
| L-leucine | 2.0 g |
| L-lysine (as acetate) | 1.4 g |
| L-methionine | 1.0 g |
| L-phenylalanine | 1.0 g |
| L-threonine | 0.5 g |
| L-tryptophan | 0.5 g |
| L-valine | 1.5 g |
| L-alanine | 0.6 g |
| L-arginine | 0.6 g |
| L-aspartic acid | 0.05 g |
| L-glutamic acid | 0.05 g |
| L-histidine | 0.5 g |
| L-proline | 0.4 g |
| L-serine | 0.2 g |
| L-tyrosine | 0.1 g |
| glycine | 0.3 g |
| L-cysteine | 0.05 g. |

While L-lysine is provided in the form of acetate, it may be given as chloride to ensure that the infusion fluid is free of acetic acid.

In addition to the above-described amino acids, the amino acid infusion fluid of the present invention may contain L-carnitine. The amino acids are not necessarily used in their free forms: They may be provided in the form of inorganic acid salts or organic acid salts. L-carnitine is preferably added at a concentration of 0.1 to 10 g/L, more preferably at a concentration of 1 to 5 g/L.

The amino acid infusion fluid of the present invention may further contain a sugar depending on the conditions of patients. While any sugar commonly used in infusion fluids may be used, reducing sugars, such as glucose, fructose and maltose, and nonreducing sugars, such as trehalose, xylitol, sorbitol and glycerol, are preferably used. Of these sugars, glucose is particularly preferred in terms of nutrition.

When the amino acid infusion fluid of the present invention is used, for example, in dialysis patients with diabetic nephropathy receiving insulin treatments, it preferably contains glucose at a concentration commonly used in dialysates for artificial kidneys (i.e., 0.05 to 0.2 w/v%). For the purpose of facilitating utilization of the supplied amino acids in protein synthesis, the amino acid infusion fluid of the present invention preferably contains glucose at a concentration of 0.75 to 3.5 w/v%. When the glucose concentration in the infusion fluid is from 0.05 to 0.2 w/v% and it is desired to facilitate utilization of the supplied amino acids in protein synthesis, patients are advised to have a nonprotein calorie intake of 500 Kcal or above for every 1.6 g of total nitrogen intake during hemodialysis.

The amino acid infusion fluid of the present invention may further contain various vitamins, including vitamin B₁, vitamin B₂, vitamin B₆, vitamin B₁₂, vitamin C, vitamin A, vitamin D, vitamin E, vitamin K, vitamin H, folic acid, pantothenic acid and nicotinic acid. Vitamins facilitate maintenance and improvement of nutritional status.

The amino acid infusion fluid of the present invention may further contain trace elements. The term "trace element" as used herein refers to a metal element that is present in the body in trace amounts but is essential to the proper functioning of the body. Among preferred trace elements are inorganic or organic salts of zinc, copper and selenium, each a metal element decreased in dialysis patients with chronic renal failure. Administration of the trace elements serves not only to prevent deficiencies of these elements, but also to facilitate protein synthesis, as well as maintenance and improvement of nutritional status. The trace elements are added to the infusion fluid to meet daily requirements.

Preferably, the amino acid infusion fluid of the present invention has a pH of 6.6 to 7.6 and an osmotic ratio to saline of about 2. The pH of the infusion fluid can be adjusted by inorganic acids, such as sodium hydroxide and hydrochloric acid, organic acids, such as acetic acid and citric acid, and gases, such as carbon dioxide.

The amino acid infusion fluid of the present invention is preferably packaged in suitable containers. When necessary, different components of the infusion fluid may be packaged in different compartments defined in a container. These compartments may be separated by separators that are designed to bring the compartments into fluid communication with each other to allow mixing of the components. It is preferred that amino acids that are unstable at high pH values, such as cysteine and tryptophan, be packaged separately from sodium bicarbonate. Amino acids are packaged separately from sodium bicarbonate when the presence of amino acids lowers the pH and, thus, the concentration of sodium bicarbonate.

The amino acid infusion fluid of the present invention is preferably packaged in a container made of a soft resin material that has high flexibility and transparency and hardly breaks when dropped onto a hard surface after storage at low temperatures. Polyolefins, a material commonly used in medical containers, are particularly preferred for this purpose. Examples of the polyolefin include polyethylene, polypropylene, poly-1-butene, poly-4-methyl-1-pentene and other polymers. The container may be made of shaping the above-describe resins by blow injection, or by inflation or deflation molding techniques. Alternatively, a pair of resin sheets may be welded along the periphery to form a pouch.

### Examples

Embodiments of the present invention will now be described with reference to examples, which are not intended to limit the scope of the invention in any way.

### Example 1: Preparation of antianemic preparation (amino acid infusion fluid)

The components shown in Table 1 below were dissolved in water for injection in the specified amounts. The solution was subjected to sterile filtration and placed in a glass vial. The vial was sealed and autoclaved to make an antianemic preparation (amino acid infusion fluid).

The preparation had a pH of 6.6 to 7.6 and an osmotic ratio of about 2.

**Table 1:**

| Components | In 200mL |
|---|---|
| L-isoleucine | 1.500g |
| L-leucine | 2.000g |
| L-lysine (as acetate) | 1.400g |
| L-methionine | 1.000g |
| L-phenylalanine | 1.000g |
| L-threonine | 0.500g |
| L-tryptophan | 0.500g |
| L-valine | 1.500g |
| L-alanine | 0.600g |
| L-arginine | 0.600g |
| L-aspartic acid | 0.050g |
| L-glutamic acid | 0.050g |
| L-histidine | 0.500g |
| L-proline | 0.400g |
| L-serine | 0.200g |
| L-tyrosine | 0.100g |
| Glycine | 0.300g |
| Total Amino Acid | 12.200g |
| Amino Acid concentration | 6.100 w/v% |
| Additives | In 200mL |
| L-cysteine | 0.050g |
| Sodium hydrogen sulfite | 0.050g |
| Glacial acetic acid (pH adjuster) | As desired |

The amino acid infusion fluid formulated according to Example 1 also serves as any of the following: A pharmaceutical preparation for reducing the dose of erythropoietin (the second aspect of the invention), a pharmaceutical preparation for controlling the serum phosphate levels (the third aspect of the invention) and a pharmaceutical preparation for suppressing protein catabolism (the fourth aspect of the invention).

### Example 2: Determination of the ability of the pharmaceutical preparation to ameliorate anemia and reduce the dose of erythropoietin

18 patients with chronic renal failure receiving hemodialysis (HD: 9 patients) or hemodiafiltration (HDF: 9 patients) were administered an exemplary antianemic preparation of the present invention to examine the ability of the preparation to ameliorate anemia and decrease the dose of erythropoietin.

### Methods

A commercially available amino acid infusion fluid "NEOAMYU (registered trademark)" (200 mL preparation, Ajinomoto Pharma Co., Ltd.) formulated according to Example 1 was used.

Necessary checkups, tests and examinations were conducted on the day of the first dialysis (Monday or Tuesday) during the week before the administration period. Starting one week after the checkups, the "NEOAMYU" preparation was continuously administered over a time period of 12 weeks and one day (the day of 37th dialysis). Specifically, one bottle (200 mL) of the preparation was infused at each dialysis session into the vein side of the dialysis circuit. The preparation was delivered over the entire course of dialysis.

Erythropoietin (EPO) was intravenously administered three times a week at each of the dialysis sessions during the week. Specifically, the dose of EPO was adjusted so that the hemoglobin (Hb) concentration was maintained in the range of 10 to 11 g/dL and the hematocrit (Ht) was maintained in the range of 30 to 33%.

### Results

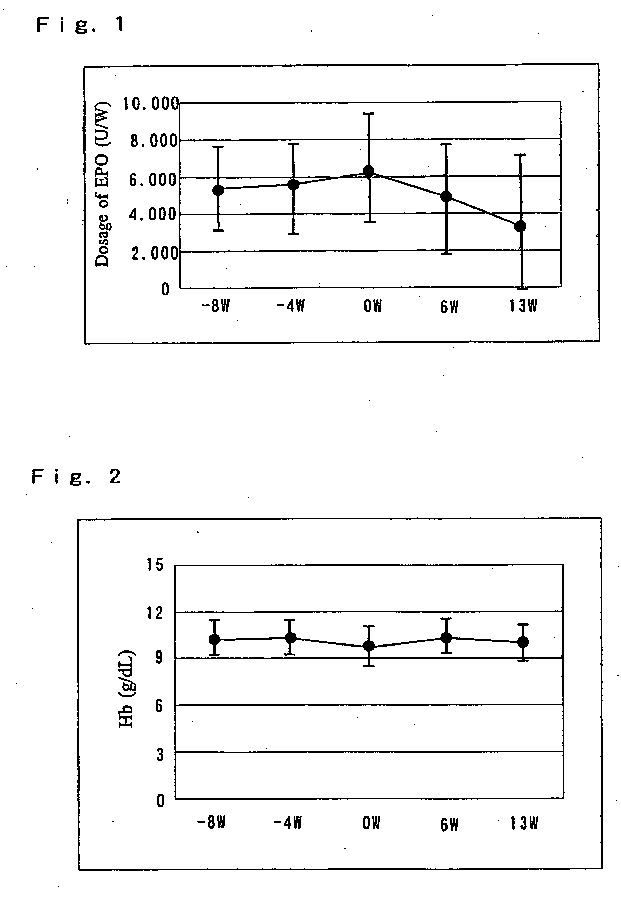

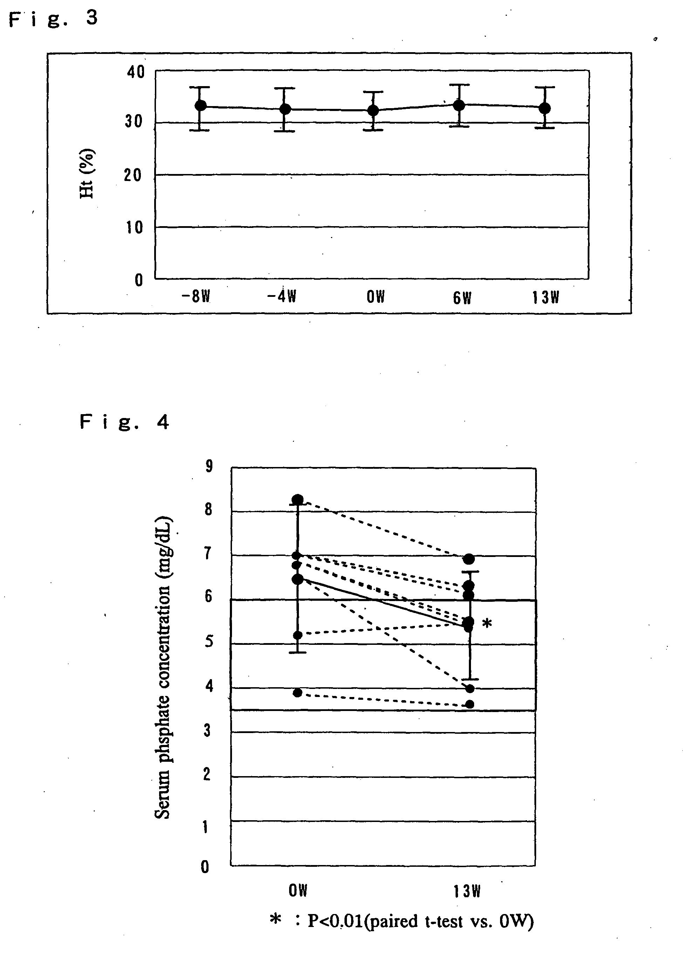

Changes in the dose of EPO are shown in Fig. 1. Changes in the Hb concentration and the Ht are shown in Figs. 2 and 3, respectively.

As can be seen from the results of these figures, the dose of EPO required to maintain the Hb concentration and the Ht was 6,125 ± 3,163.7 units/week before administration of "NEOAMYU" product (Week 0). 6 weeks after the start of administration (Week 6), the dose was decreased to 4,875 ± 3,111.0 units/week. On Week 13, the dose was significantly decreased to 3,375 ± 3,557.6 units/week (p < 0.01 on Wilcoxon Matched-Pair Signed-Rank Test).

The Hb concentration and Ht were kept stable during the administration of the product.

These observations indicate that the amino acid infusion fluid of the present invention can effectively ameliorate anemia in dialysis patients, thus reducing the dose of EPO administered to these patients.

### Example 3: Determination of the ability of the pharmaceutical preparation to control the serum phosphate levels

8 patients with chronic renal failure receiving hemodialysis (HD) were administered an exemplary serum phosphate-controlling preparation of the present invention to examine the changes in the serum phosphate levels.

### Methods

A commercially available amino acid infusion fluid "NEOAMYU" (200 mL preparation, Ajinomoto Pharma Co., Ltd.) formulated according to Example 1 was used.

Necessary checkups, tests and examinations were conducted on the day of the first dialysis (Monday or Tuesday) during the week before the administration period. Starting one week after the checkups, the "NEOAMYU" product was continuously administered over a time period of 12 weeks and one day (the day of 37th dialysis). Specifically, one bottle (200 mL) of the preparation was infused at each dialysis session into the vein side of the dialysis circuit. The preparation was delivered over the entire course of dialysis. The serum phosphate levels were measured on Week 0 (before administration) and on Week 13.

### [Results]

The results are shown in Fig. 4.

As can be seen from the results of the figure, the serum phosphate level significantly decreased from 6.48 ± 1.752 mg/dL on Week 0 (before administration of the pharmaceutical preparation of the present invention) to 5.41 ± 1.253 mg/dL (p < 0.01 vs. Week 0 on Paired t-test) on Week 13.

The serum phosphate level in patients who had an initial serum phosphate level within the target range (3.5 to 6.0 mg/dL) remained within the range. In comparison, the blood phosphate level decreased significantly in patients who had an initial serum phosphate level of higher than 6.0 mg/dL.

The product of the serum phosphate level and the serum calcium level (Ca x P) was 56.7 ± 13.88 (mg/dL)² on Week 0 (before administration) and decreased significantly (p < 0.01 vs. Week 0 on Paired t-test) to 48.5 ± 13.89 (mg/dL)² on Week 13, which was within the target range (55 or less).

These results indicate that the pharmaceutical preparation of the present invention can effectively control the serum phosphate levels in dialysis patients.

### Example 4: Changes in plasma 3-m-His/taurine levels and concentrations of 3-m-His/taurine in dialysate

9 patients with chronic renal failure receiving hemodialysis (HD) were administered an exemplary catabolism-suppressing preparation of the present invention to examine the changes in plasma 3-m-His levels, 3-m-His concentrations in dialysate, plasma taurine levels and taurine concentrations in dialysate.

### Methods

A commercially available amino acid infusion fluid "NEOAMYU" (200 mL preparation, Ajinomoto Pharma Co., Ltd.) formulated according to Example 1 was used.

Necessary checkups, tests and examinations were conducted on the day of the first dialysis (Monday or Tuesday) during the week before the administration period. Starting one week after the checkups, the "NEOAMYU" product was continuously administered over a time period of 12 weeks and one day (the day of 37th dialysis). Specifically, one bottle (200 mL) of the preparation was infused at each dialysis session into the vein side of the dialysis circuit. The preparation was delivered over the entire course of dialysis. The plasma 3-m-His levels, 3-m-His concentrations in dialysate, plasma taurine levels and taurine concentrations in dialysate were measured on Week 0 (before administration), on Week 6 and on Week 13.

### Results

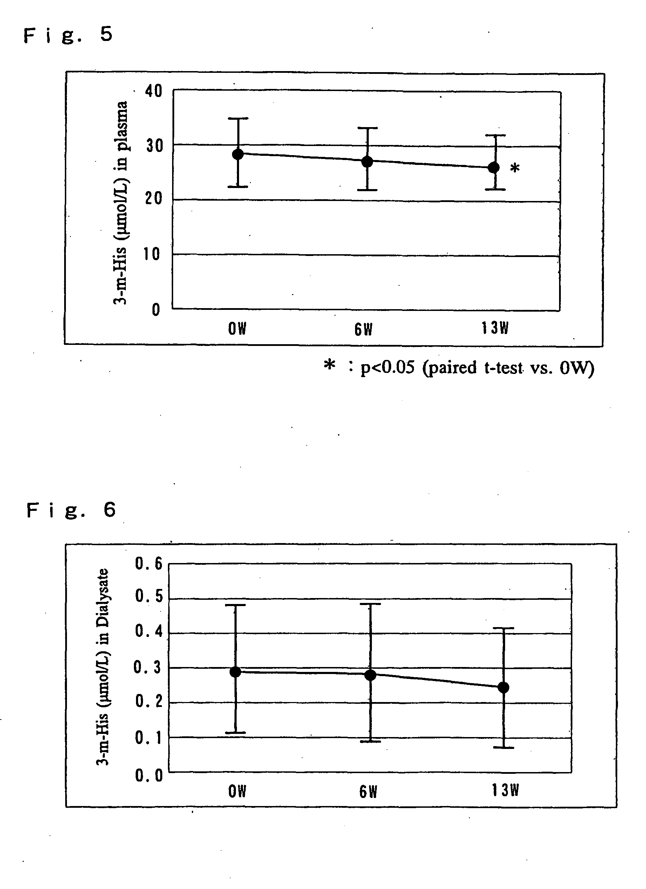

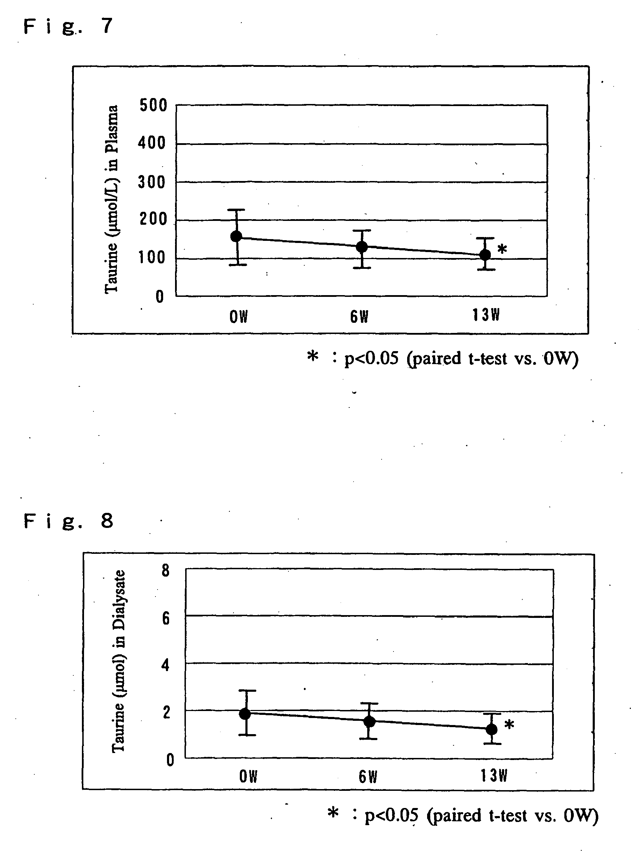

The plasma 3-m-His levels and the concentrations of 3-m-His in dialysate are shown in Fig. 5 and Fig. 6, respectively. The plasma taurine levels and the concentrations of taurine in dialysate are shown in Fig. 7 and Fig. 8, respectively.

As can be seen from the results of the figure, the plasma 3-m-His level, a common index of myolysis, was 28.04 ± 6.255 µmol/L on Week 0 (before administration of the pharmaceutical preparation of the present invention) and decreased to 27.32 ± 6.254 µmol/L on Week 6 and significantly to 26.56 ± 5.27 µmol/L (p < 0.05 vs. Week 0 on Paired t-test).

The 3-m-His concentration in dialysate, which was 0.29 ± 0.19 µmol on Week 0 and 0.29 ± 0.20 µmol on Week 6, also decreased to 0.24 ± 0.18 µmol on Week 13, when the plasma level showed a significant decrease. Thus, the 3-m-His concentration in dialysate indicated the presence of myolysis on Week 13.

In comparison, the plasma taurine level decreased from 156.08 ± 72.74 µmol/L on Week 0 (before administration of the pharmaceutical preparation of the present invention) to 127.80 ± 50.90 µmol/L on Week 6 and significantly to 107.44 ± 42.30 µmol/L on Week 13 (p < 0.05 vs. Week 0 on Paired t-test).

The concentration of taurine in dialysate also significantly decreased from 1.92 ± 1.13 µmol on Week 0 to 1.45 ± 0.78 µmol on Week 6 and to 1.20 ± 0.65 µmol on Week 13 (p < 0.05 vs. Week 0 on Paired t-test), when the plasma level showed a significant decrease.

Thus, it has been demonstrated that changes observed for taurine were more significant than those observed for 3-m-His, suggesting that taurine serves as a more sensitive index of protein catabolism than 3-m-His in dialysis patients.

### Example 5: Determination of the blood amino acid levels in dialysis patients

The blood amino acid levels in dialysis patients undergoing the above-described tests were measured.

9 patients with chronic renal failure receiving hemodialysis (HD) were administered the amino acid infusion fluid "NEOAMYU" (Ajinomoto Pharma Co., Ltd.). The blood amino acid levels and amino acid concentrations in dialysate were measured.

Necessary checkups, tests and examinations were conducted on the day of the first dialysis (Monday or Tuesday) during the week before the administration period. Starting one week after the checkups, the "NEOAMYU" preparation was continuously administered over a time period of 12 weeks and one day (the day of 37th dialysis). Specifically, one bottle (200 mL) of the preparation was infused at each dialysis session into the vein side of the dialysis circuit. The preparation was delivered over the entire course of dialysis.

The total amino acid concentration in plasma was decreased by half, from about 4000 µmol/L before dialysis to about 2000 µmol/L after dialysis. The total amino acid (TAA) concentration showed a slight increase during dialysis by the administration of "NEOAMYU". When essential amino acids (EAA) and non-essential amino acids (NEAA) were considered separately, however, the EAA concentration remained substantially the same throughout dialysis. This observation suggests that the continuous infusion of ""NEOAMYU" (200 mL preparation) prevents the decrease in the EAA concentration.

In comparison, the NEAA concentration remained substantially unchanged during dialysis, and the dominance of EAAs (NEAAs: EAA (E/N) = 3.21) was maintained during dialysis.

As it turned out, the amount of amino acids in dialysate, which was 6 g before administration of "NEOAMYU" (control), increased by about 3 g on Week 6 by the administration of "NEOAMYU" and returned to the initial level on Week 13.

It is generally believed that amino acids administered during dialysis will be immediately removed by dialysis. However, these results, together with the fact that "NEOAMYU", the amino acid infusion fluid of the present example, contains 11.8g of amino acid, suggest that the amino acid infusion fluid continuously infused during dialysis keeps amino acids, in particular essential amino acids, from being eliminated from the body and serves to maintain the blood amino acid levels.

Thus, it is concluded that the method using the infusion fluid of the present invention improves various conditions of dialysis patients and, as a result, anemia can be ameliorated and protein catabolism can be suppressed in these patients.

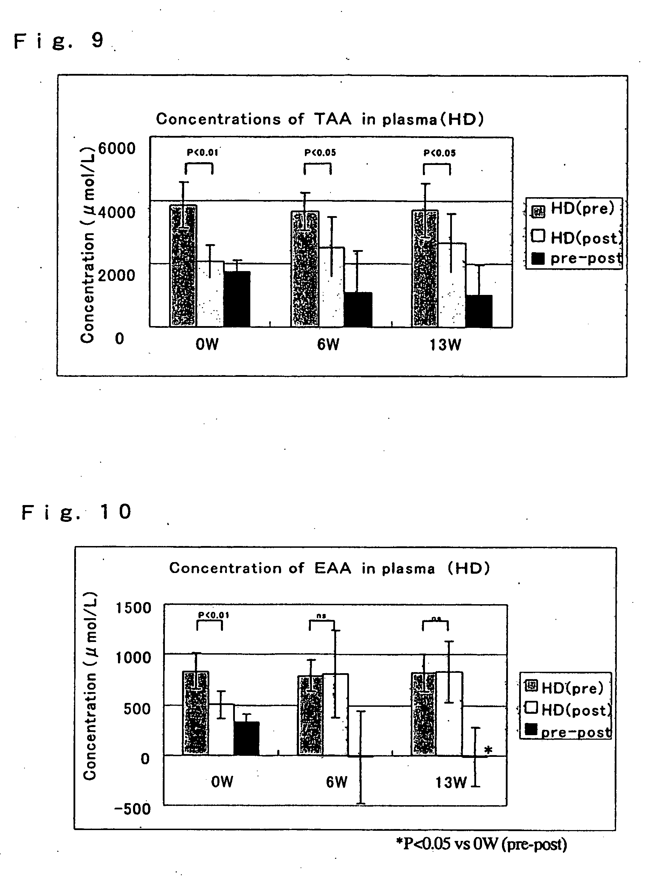

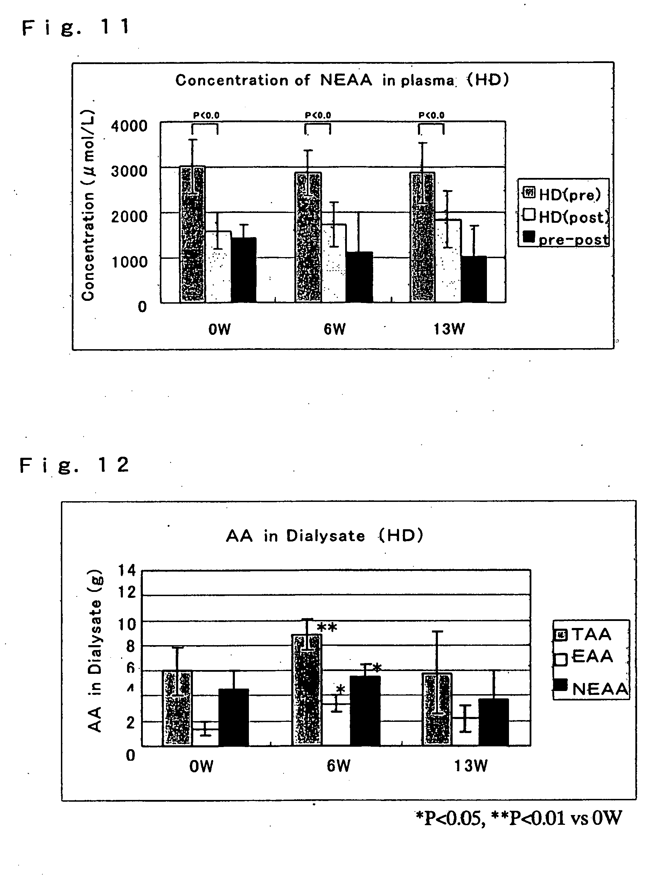

These results are collectively shown in Fig. 9 through Fig. 12.

The plasma concentrations of essential amino acids and non-essential amino acids are shown in Tables 2 through 4 below.

Table 2 shows plasma concentrations of essential amino acids before administration of the amino acid infusion fluid (Week 0).

Table 3 shows plasma concentrations of essential amino acids after administration of the amino acid infusion fluid (Week 13).

**Table 2:**

| | Standards | | Administration | | Difference | Dialysate | Rate of removal (%) | Dialysate/ before administration |
|---|---|---|---|---|---|---|---|---|
| | | | Before | After | | | | |
| | Min. | Max. | Average | | Average | Average | | |
| Threonine | 74.2 | 216.1 | 131.3 | 68.3 | 63.0 | 1.87 | 48.0 | 0.014 |
| Valine | 156.2 | 360.4 | 188.9 | 105.6 | 83.3 | 2.98 | 44.1 | 0.016 |
| methionine | 15.5 | 38.6 | 31.2 | 16.5 | 14.8 | 0.49 | 47.3 | 0.016 |
| isoleucine | 37.0 | 100.4 | 71.7 | 47.4 | 24.3 | 1.19 | 33.9 | 0.017 |
| Leucine | 74.2 | 169.1 | 106.5 | 81.2 | 25.3 | 1.77 | 23.8 | 0.017 |
| phenylalanine | 43.5 | 79.8 | 73.8 | 51.5 | 22.3 | 1.36 | 30.2 | 0.018 |
| Lysine | 125.7 | 281.9 | 208.2 | 110.9 | 97.3 | 2.82 | 46.7 | 0.014 |
| tryptophan | 36.2 | 79.3 | 22.1, | 21.5 | 0.7 | 0.57 | 3.0 | 0.020 |
| Histidine | 63.0 | 105.2 | 101.9 | 57.7 | 44.2 | 1.49 | 43.3 | 0.015 |

**Table 3:**

| | Standards | | Administration | | Difference | Dialysate | Rate of removal (%) | Dialysate/ before administration |
|---|---|---|---|---|---|---|---|---|
| | | | Before | After | | | | |
| | Min. | Max. | Average | | Average | Average | | |
| threonine | 74.2 | 216.1 | 143.8 | 108.9 | 34.9 | 24.3 | 24.3 | 0.169 |
| valine | 156.2 | 360.4 | 183.0 | 195.8 | -12.8 | 43.4 | -7.0 | 0.237 |
| methionine | 15.5 | 38.6 | 28.1 | 42.2 | -14.1 | 11.2 | -50.1 | 0.399 |
| isoleucine | 37.0 | 100.4 | 59.4 | 81.7 | -22.3 | 20.6 | -37.6 | 0.347 |
| leucine | 74.2 | 169.1 | 99.6 | 137.4 | -37.8 | 69.3 | -38.0 | 0.696 |
| phenylalanine | 43.5 | 79.8 | 64.3 | 77.4 | -13.1 | 15.1 | -20.4 | 0.235 |
| lysine | 125.7 | 281.9 | 214.7 | 151.0 | 63.7 | 25.0 | 29.7 | 0.117 |
| tryptophan | 36.2 | 79.3 | 23.7 | 34.3 | -10.5 | 6.6 | -44.4 | 0.276 |
| histidine | 63.0 | 105.2 | 109.5 | 75.6 | 33.9 | 14.4 | 31.0 | 0.132 |

As can be seen from a comparison between Tables 2 and 3, continuous infusion of the amino acid infusion fluid of the present invention prevents the decrease in the plasma concentrations of essential amino acids.

Table 4 shows plasma concentrations of non-essential amino acids before administration of the amino acid infusion fluid (Week 0).

Table 5 shows plasma concentrations of non-essential amino acids after administration of the amino acid infusion fluid (Week 13).

**Table 4:**

| | Standards | | Administration | | Difference | Dialysate | Rate of removal (%) | Dialysate/ before administration |
|---|---|---|---|---|---|---|---|---|
| | | | Before | After | | | | |
| | Min. | Max. | Average | | Average | Average | | |
| aspartic acid | 0.0 | 7.2 | 5.0 | 3.3 | 1.6 | - | 32.7 | - |
| asparagine | 43.8 | 90.6 | 74.5 | 45.5 | 29.0 | 2.03 | 38.9 | 0.027 |
| serine | 91.5 | 186.4 | 122.7 | 72.7 | 50.0 | 1.84 | 40.7 | 0.015 |
| glutamic acid | 12.2 | 82.7 | 70.1 | 79.3 | -9.2 | 1.33 | -13.1 | 0.019 |
| glutamine | 418.0 | 739.8 | 601.5 | 393.9 | 207.5 | 9.78 | 34.5 | 0.016 |
| proline | 71.3 | 373.0 | 299.1 | 163.4 | 135.6 | 7.11 | 45.4 | 0.024 |
| glycine | 140.4 | 427.3 | 366.5 | 202.4 | 164.1 | 5.34 | 44.8 | 0.015 |
| alanine | 258.8 | 615.2 | 456.8 | 204.5 | 252.3 | 5.86 | 55.2 | 0.013 |
| histidine | 4.7 | 34.8 | 111.7 | 27.1 | 84.6 | 1.65 | 75.7 | 0.015 |
| threonine | 38.4 | 89.4 | 42.1 | 26.0 | 16.0 | 0.54 | 38.1 | 0.013 |
| arginine | 31.8 | 149.5 | 74.2 | 53.3 | 20.9 | 1.41 | 28.1 | 0.019 |
| taurine | 46.4 | 128.2 | 224.8 | 72.8 | 152.0 | 2.73 | 67.6 | 0.012 |

**Table 5:**

| | Standards | | Administration | | Difference | Dialysate | Rate of removal (%) | Dialysate/ before administration |
|---|---|---|---|---|---|---|---|---|
| | | | Before | After | | | | |
| | Min. | Max. | Average | | Average | Average | | |
| aspartic acid | 0.0 | 7.2 | 4.6 | 4.2 | 0.4 | 2.1 | 9.0 | 0.459 |
| asparagine | 43.8 | 90.6 | 66.3 | 44.6 | 21.7 | 9.8 | 32.7 | 0.147 |
| serine | 91.5 | 186.4 | 118.4 | 83.4 | 34.7 | 11.2 | 29.3 | 0.094 |
| glutamic acid | 12.2 | 82.7 | 80.2 | 91.5 | -11.3 | 14.2 | -14.1 | 0.177 |
| glutamine | 418.0 | 739.8 | 565.5 | 411.8 | 153.7 | 105.3 | 27.2 | 0.186 |
| proline | 71.3 | 373.0 | 254.6 | 204.5 | 50.1 | 39.1 | 19.7 | 0.153 |
| glycine | 140.4 | 427.3 | 347.5 | 219.7 | 127.9 | 35.6 | 36.8 | 0.102 |
| alanine | 258.8 | 615.2 | 419.1 | 282.8 | 136.3 | 67.7 | 32.5 | 0.162 |
| histidine | 4.7 | 34.8 | 111.7 | 22.5 | 89.2 | 11.9 | 79.9 | 0.106 |
| threonine | 38.4 | 89.4 | 37.2 | 30.8 | 6.3 | 3.6 | 17.1 | 0.096 |
| arginine | 31.8 | 149.5 | 90.0 | 44.5 | 45.5 | 20.6 | 50.6 | 0.229 |
| taurine | 46.4 | 128.2 | 181.9 | 76.2 | 120.6 | 13.9 | 66.3 | 0.077 |

As can be seen from a comparison between Tables 4 and 5, no significant differences were observed between the concentrations of non-essential amino acids measured before administration and those measured after administration. Thus, the dominance of essential amino acids (NEAAs: EAA (E/N) = 3.21) was maintained.

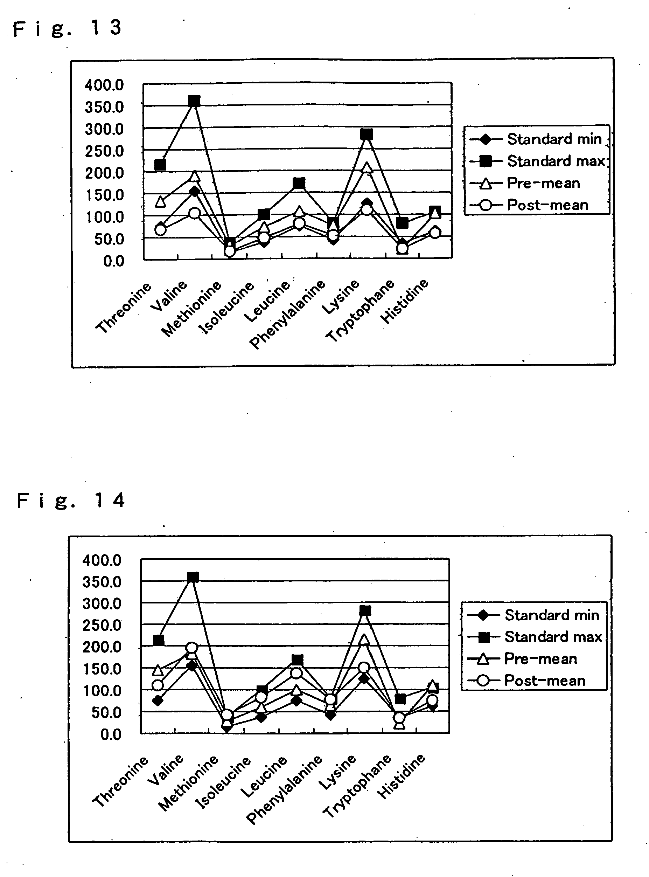

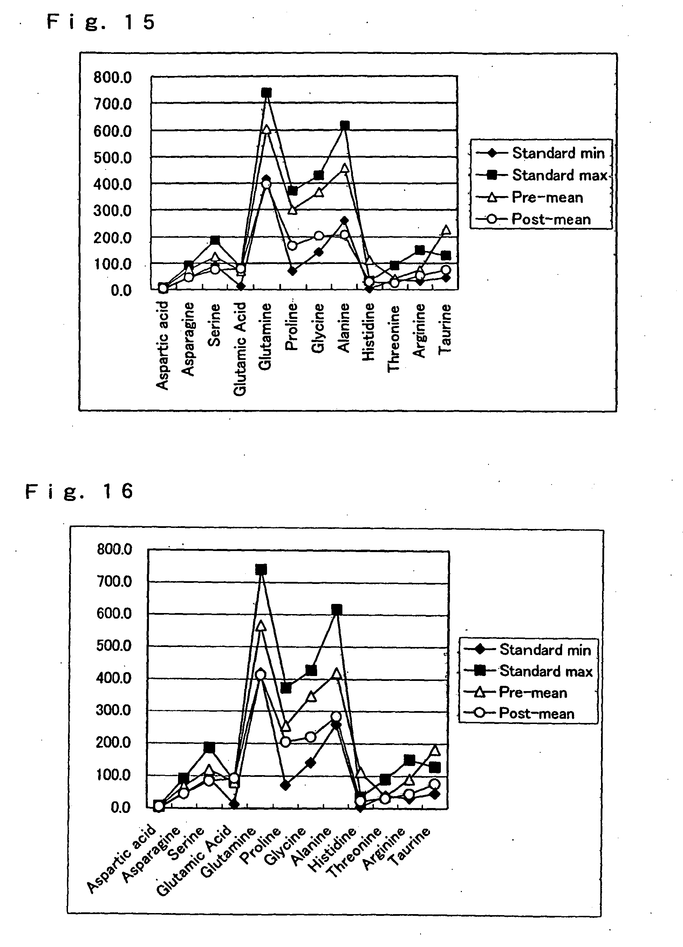

The plasma aminograms based on the plasma amino acid concentrations are shown in Figs. 13 through 16.

These results demonstrate that the amino acid infusion fluid of the present invention maintains the plasma amino acid levels in dialysis patients in a well-balanced manner throughout the administration period.

As set forth, the amino acid infusion fluid of the present invention, which serves as a pharmaceutical preparation for ameliorating anemia in dialysis patients, can be continuously administered to patients with chronic renal failure receiving hemodialysis to suppress protein degradation and maintain/improve nutritional status of such patients. As a result, anemia can be ameliorated and, consequently, the dose of erythropoietin administered during dialysis can be reduced in these patients.

The pharmaceutical preparation of the present invention for controlling the serum phosphate levels can suppress protein degradation and maintain/improve nutritional status of patients with chronic renal failure receiving hemodialysis. As a result, the serum phosphate levels in such patients can be controlled within a predetermined range. Specifically, the serum phosphate levels in patients with chronic renal failure can be decreased to fall within a predetermined range when they are too high or maintained within that range when they are already in the range.

In addition, the amino acid infusion fluid of the present invention, which also serves as a pharmaceutical preparation for suppressing protein catabolism, can be continuously administered to patients with chronic renal failure receiving hemodialysis to prevent muscle weakening and a decrease in cardiovascular functions and decreased QOL of such patients, symptoms resulting from hemodialysis. The amino acid infusion fluid of the present invention can prevent or alleviate these symptoms even when patients are receiving long-term hemodialysis.

Thus, the amino acid infusion fluid of the present invention can be used to avoid risks of complications in patients with chronic renal failure receiving hemodialysis and to thereby improve quality of life (QOL) and prognosis of such patients. The present invention therefore is of significant medical importance.

## Claims

1. An infusion fluid for use in treating anemia in a patient receiving dialysis, the fluid containing at least one essential amino acid, and being administered to the patient by continuous infusion.

2. The fluid for use in treating anemia in a patient receiving dialysis according to claim 1, having an amino acid composition comprising at least L-isoleucine, L-leucine, L-lysine, L-methionine, L-phenylalanine, L-threonine, L-tryptophan, L-valine, L-alanine, L-arginine, L-aspartic acid, L-glutamic acid, L-histidine, L-proline, L-serine, L-tyrosine, glycine and L-cysteine, wherein a ratio of essential amino acids to non-essential amino acids is 2.5 or higher.

3. The infusion fluid for use in treating anemia in a patient receiving dialysis according to claim 1 or 2, having the following amino acid composition:
| | |
|---|---|
| L-isoleucine | 5.0 - 10.0 g/L |
| L-leucine | 8.0 - 12.0 g/L |
| L-lysine (as acetate) | 4.5 - 10.5 g/L |
| L-methionine | 3.5 - 6.5 g/L |
| L-phenylalanine | 3.5 - 6.5 g/L |
| L-threonine | 1.8 - 3.3 g/L |
| L-tryptophan | 1.8 - 3.3 g/L |
| L-valine | 5.0 - 10.0 g/L |
| L-alanine | 1.5 - 4.5 g/L |
| L-arginine | 1.5 - 4.5 g/L |
| L-aspartic acid | 0.2 - 0.4 g/L |
| L-glutamic acid | 0.2 - 0.4 g/L |
| L-histidine | 1.8 - 3.3 g/L |
| L-proline | 1.0 - 3.0 g/L |
| L-serine | 0.5 - 1.5 g/L |
| L-tyrosine | 0.3 - 0.7 g/L |
| glycine | 1.2 - 1.7 g/L |
| L-cysteine | 0.2 - 0.4 g/L. |

4. The infusion fluid for use in treating anemia in a patient receiving dialysis according to claim 1, 2 or 3, wherein the pharmaceutical preparation is continuously infused by means of a pump into a vein side of a dialysis circuit.

5. An infusion fluid for use in treating anemia in a patient receiving dialysis, according to any one of claims 1 to 4, wherein the fluid is also for use in reducing the dose of erythropoietin in a patient receiving dialysis.

6. An infusion fluid for use in treating anemia in a patient receiving dialysis, according to any one of claims 1 to 4, wherein the fluid is, additionally, for use in controlling serum phosphate levels.

7. An infusion fluid for use in treating anemia in a patient receiving dialysis, according to any one of claims 1 to 4, wherein the fluid is, additionally, for suppressing protein catabolism.

## Patentansprüche

1. Infusionsfluid für die Verwendung in der Behandlung von Anämie in einem Dialysepatienten, wobei das Fluid mindestens eine essentielle Aminosäure enthält und dem Patienten durch kontinuierliche Infusion verabreicht wird.

2. Fluid für die Verwendung in der Behandlung von Anämie in einem Dialysepatienten nach Anspruch 1, das eine Aminosäurezusammensetzung enthält, die mindestens L-Isoleucin, L-Leucin, L-Lysin, L-Methionin, L-Phenylalanin, L-Threonin, L-Tryptophan, L-Valin, L-Alanin, L-Arginin, L-Asparaginsäure, L-Glutaminsäure, L-Histidin, L-Prolin, L-Serin, L-Tyrosin, Glycin und L-Cystein umfasst, wobei das Verhältnis von essentiellen Aminosäuren zu nicht-essentiallen Aminosäuren 2,5 oder höher ist.

3. Infusionsfluid für die Verwendung in der Behandlung von Anämie in einem Dialysepatienten nach Anspruch 1 oder 2 mit der folgenden Aminosäurezusammensetzung:
| |
|---|
| L-Isoleucin 5,0 - 10,0 g/L |
| L-Leucin 8,0 - 12,0 g/L |
| L-Lysin (als Acetat) 4,5 - 10,5 g/L |
| L-Methionin 3,5 - 6,5 g/L |
| L-Phenylalanin 3,5 - 6,5 g/L |
| L-Threonin 1,8 - 3,3 g/L |
| L-Tryptophan 1,8 - 3,3 g/L |
| L-Valin 5,0 - 10,0 g/L |
| L-Alanin 1,5 - 4,5 g/L |
| L-Arginin 1,5 - 4,5 g/L |
| L-Asparaginsäure 0,2 - 0,4 g/L |
| L-Glutaminsäure 0,2 - 0,4 g/L |
| L-Histidin 1,8 - 3,3 g/L |
| L-Prolin 1,0 - 3,0 g/L |
| L-Serin 0,5 - 1,5 g/L |
| L-Tyrosin 0,3 - 0,7 g/L |
| Glycin 1,2 - 1,7 g/L |
| L-Cystein 0,2 - 0,4 g/L. |

4. Infusionsfluid für die Verwendung in der Behandlung von Anämie in einem Dialysepatienten nach Anspruch 1, 2 oder 3, wobei die Arzneimittelzubereitung mit Hilfe einer Pumpe kontinuierlich in eine Venenseite eines Dialysekreislaufs infundiert wird.

5. Infusionsfluid für die Verwendung in der Behandlung von Anämie in einem Dialysepatienten nach einem der Ansprüche 1 bis 4, wobei das Fluid auch für die Verwendung zur Verringerung der Dosis von Erythropoietin in einem Dialysepatienten vorgesehen ist.

6. Infusionsfluid für die Verwendung in der Behandlung von Anämie in einem Dialysepatienten nach einem der Ansprüche 1 bis 4, wobei das Fluid zusätzlich für die Verwendung bei der Kontrolle von Serumphosphatspiegeln vorgesehen ist.

7. Infusionsfluid für die Verwendung in der Behandlung von Anämie in einem Dialysepatienten nach einem der Ansprüche 1 bis 4, wobei das Fluid zusätzlich für die Unterdrückung des Proteinkatabolismus vorgesehen ist.

## Revendications

1. Liquide de perfusion utilisé dans le traitement de l'anémie chez un patient sous dialyse, le liquide contenant au moins un acide aminé essentiel, et étant administré au patient par perfusion continue.

2. Liquide utilisé dans le traitement de l'anémie chez un patient sous dialyse selon la revendication 1, ayant une composition d'acides aminés comprenant au moins de la L-isoleucine, de la L-leucine, de la L-lysine, de la L-méthionine, de la L-phénylalanine, de la L-thréonine, du L-tryptophane, de la L-valine, de la L-alanine, de la L-arginine, de l'acide L-aspartique, de l'acide L-glutamique, de la L-histidine, de la L-proline, de la L-sérine, de la L-tyrosine, de la glycine et de la L-cystéine, dans lequel le rapport des acides aminés essentiels aux acides aminés non essentiels est supérieur ou égal à 2,5.

3. Liquide de perfusion utilisé dans le traitement de l'anémie chez un patient sous dialyse selon la revendication 1 ou 2, ayant la composition d'acides aminés suivante :
| | |
|---|---|
| L-isoleucine | 5,0 - 10,0 g/l |
| L-leucine | 8,0 - 12,0 g/l |
| L-lysine (sous forme d'acétate) | 4,5 - 10,5 g/l |
| L-méthionine | 3,5 - 6,5 g/l |
| L-phénylalanine | 3,5 - 6,5 g/l |
| L-thréonine | 1,8 - 3,3 g/l |
| L-tryptophane | 1,8 - 3,3 g/l |
| L-valine | 5,0 - 10,0 g/l |
| L-alanine | 1,5 - 4,5 g/l |
| L-arginine | 1,5 - 4,5 g/l |
| Acide L-aspartique | 0,2 - 0,4 g/l |
| Acide L-glutamique | 0,2 - 0,4 g/l |
| L-histidine | 1,8 - 3,3 g/l |
| L-proline | 1,0 - 3,0 g/l |
| L-sérine | 0,5 - 1,5 g/l |
| L-tyrosine | 0,3 - 0,7 g/l |
| glycine | 1,2 - 1,7 g/l |
| L-cystéine | 0,2 - 0,4 g/l |

4. Liquide de perfusion utilisé dans le traitement de l'anémie chez un patient sous dialyse selon la revendication 1, 2 ou 3, dans lequel la préparation pharmaceutique est perfusée en continu au moyen d'une pompe côté veine d'un circuit de dialyse.

5. Liquide de perfusion utilisé dans le traitement de l'anémie chez un patient sous dialyse, selon l'une quelconque des revendications 1 à 4, le liquide étant également utilisé dans la réduction de la dose d'érythropoïétine chez un patient sous dialyse.

6. Liquide de perfusion utilisé dans le traitement de l'anémie chez un patient sous dialyse, selon l'une quelconque des revendications 1 à 4, le liquide étant en outre utilisé dans la limitation des niveaux de phosphate sérique.

7. Liquide de perfusion utilisé dans le traitement de l'anémie chez un patient sous dialyse, selon l'une quelconque des revendications 1 à 4, le liquide étant en outre destiné à la suppression du catabolisme protéique.
